# EUROPEAN PATENT SPECIFICATION

(11) **EP 2 914 755 B1**
(45) Date of publication and mention of the grant of the patent: **30.08.2017**
(21) Application number: 14796564.4
(22) Date of filing: 02.10.2014
(51) Int. Cl.: C12R 1/865, C12G 3/02

(54) **METHOD FOR HONEY WORT HIGH-SUGAR ALCOHOL FERMENTATION**
VERFAHREN ZUR GÄRUNG VON HONIGMAISCHE MIT HOHER ZUCKERKONZENTRATION
PROCÉDÉ POUR LA FERMENTATION ALCOOLIQUE À HAUTE TENEUR EN SUCRE DE MOÛT DE MIEL

(30) Priority: 30.12.2013 PL 40671813
(43) Date of publication of application: 09.09.2015
(73) Proprietor: Instytut Biotechnologii Przemyslu Rolno-Spozywczego, 02-532 Warszawa (PL)
(72) Inventor: MISIEWICZ, Anna, 02-156 Warszawa (PL); WETOSZKA, Urszula, 03-130 Warszawa (PL); SPERA, Maria, 05-515 Mysiadlo (PL); CIESLAK, Hanna, 05-270 Marki (PL); TEREBIENIEC, Agata, 04-133 Warszawa (PL); KIELISZEK, Marek, 21-010 Leczna (PL)
(74) Representative: Witek, Rafal
(86) International application number: PCT/PL2014/000111
(87) International publication number: WO 2015/102500

(56) References cited:
- MENDES-FERREIRA A ET AL: "Optimization of honey-must preparation and alcoholic fermentation by Saccharomyces cerevisiae for mead production", INTERNATIONAL JOURNAL OF FOOD MICROBIOLOGY, ELSEVIER BV, NL, vol. 144, no. 1, 15 November 2010 (2010-11-15), pages 193-198, XP027481281, ISSN: 0168-1605, DOI: 10.1016/J.IJFOODMICRO.2010.09.016 [retrieved on 2010-09-24]
- PEREIRA A P ET AL: "Mead production: Selection and characterization assays of Saccharomyces cerevisiae strains", FOOD AND CHEMICAL TOXICOLOGY, PERGAMON, GB, vol. 47, no. 8, 1 August 2009 (2009-08-01) , pages 2057-2063, XP026337024, ISSN: 0278-6915, DOI: 10.1016/J.FCT.2009.05.028 [retrieved on 2009-05-27]
- PEREIRA A P ET AL: "High-cell-density fermentation ofSaccharomyces cerevisiaefor the optimisation of mead production", FOOD MICROBIOLOGY, vol. 33, no. 1, 25 September 2012 (2012-09-25), pages 114-123, XP028956814, ISSN: 0740-0020, DOI: 10.1016/J.FM.2012.09.006

## Description

The subject of the invention is a method for carrying out honey wort high-sugar alcohol fermentation with the use of an appropriate strain of yeast and dry yeast preparation that guarantees high survivability and retention of process properties. The invention belongs to the field of biotechnology of alcoholic beverages, and in particular mead.

Mead is an alcoholic beverage similar to wine obtained via the process of alcohol fermentation of honey diluted with water, the so called honey wort.

The production of meads is no different from the manufacture of fruit wines with the exception of the use of the basic raw material, that not being fruit but natural honey. The production of meads includes: honey wort preparation, cooking and seasoning the wort and its fermentation and maturing of a young mead. Meads may be classified in accordance with the manner of preparing the wort, degree to which the wort is diluted with water and the way of wort seasoning.

*Saccharomyces cerevisiae* yeast species is a typical premium yeast used in wine production. It is characterised by a high production of aromatic compounds during fermentation and are resistant to temperature and depending on the type they propagate in temperatures from 8 to 15°C, from 15 to 25°C and from 15 to 35°C. The yeast is used in the production of red and white wines. Their downside is sensitivity to high alcohol and sugar concentrations as well as high nitrogen demand. For fermentation of musts with high sugar content yeasts from the *Saccharomyces bayanus* species are often used. This yeast usually attenuate quickly, are resistant to high alcohol and sugar concentrations, have relatively small requirements in terms of nitrogen and are resistant to low temperatures. However, the obtained wine has little aroma and an increased SO₂ content.

Yeast used for the production of meads apart from the properties required for wine yeasts, i.e. preliminary fermentation and fermentation, high real degree fermentation of sugars, short generation time, additionally they should be resistant to high osmotic pressure caused by high primary extract and should exhibit resistance to formic acid occurring with the fermentation of unboiled wort meads.

The ability of *Saccharomyces cerevisiae* yeast to grow and ferment in osmophilic worts, i.e. those with a high concentration of substrates depends on the concentration of intercellular carbohydrates: trehalose and glycogen.

Together with the increased of osmotic pressure in the environment, e.g. concentrated worts with density between 25 to 30° Balling, some strains of yeast exhibit increased trehalose synthesis from 0.1% to 8% and even up to 12% in dry mass, which ensures intensive cell growth and increases the efficiency of biomass from sugar. Unfortunately osmophilic properties of yeasts acquired due to increased intracellular trehalose content gradually decrease with storage time.

Fermentation of high extract worts and mashes and in particular honey meads of Dwójniak and Trojniak is difficult due to the high concentration of sugars in the wort and the associated high osmotic pressure. During fermentation of highly concentrated environments often the desired alcohol content is not achieved and the volatile acidity increases unfavourably. In particular this is associated with high osmotic pressure of solutions, toxic effects of alcohol and other yeast metabolism products.

The process efficiency in all biotechnological processes depends on the quantity and quality of used microorganisms. On that account as well as taking into consideration maximum reduction of auxiliary operations, more and more often in place of pure starter cultures, appropriate microorganism preparations are distributed and used directly in the production. Due to a need of transport, and to ensure shelf life they come in dry form and are described as dried or instant.

Water in yeast cells acts as enzyme, nutritional substance and colloids solvent. Drying microorganisms causes it to be removed from an environment where biochemical reactions take place. As a result of dehydration structural changes on biopolymer, cellular walls, organs and entire cells occur. These processes change microorganism resistance to external factors such as pH, temperature, pressure and viscosity. Microorganism may die as a result of damaging cell membrane, protein thermal denaturation, water dissolved substance transport destabilisation on increaser in environmental viscosity. Microorganism physiological resistance during the drying process is associated with adaptive processes, physical resistance associated with the structure of particular microorganism. There is a critical moisture content, and if exceeded it causes dehydrative inactivity of microorganisms and it's progress depends on biomass humidity. The removal of water during the drying process causes protoplasm proteins to transform from hydrosol to hydrogel. This transformation occurs together with water extraction for a cell and leads to temporary, but reversible stoppage of life functions of the system - anabiosis to a degree dependant on the amount of water remaining.

The aim of *Mendes-Ferreira A et al. (2010)* studies was to optimize mead production through the use of an appropriate honey-must formulation to improve yeast performance alcoholic fermentation and thereby obtain a high quality product. Honey-must was centrifuged to reduce insoluble solids, pasteurized at 65°C for 10 min, and then subjected to different conditions: nitrogen supplementation and addition of organic acids. Although the addition of diammonium phosphate (DAP) reduced fermentation length, it did not guarantee the completeness of the fermentation process, suggesting that other factors could account for the reduced yeast activity in honey-must fermentations. The results suggested that the reduced yeast fermentative ability, and thereby increased risk of delayed or arrested fermentations, is due to factors other than nitrogen limitation in honey-musts (Mendes-Ferreira A et al. Optimization of honey-must preparation and alcoholic fermentation by Saccharomyces cerevisiae for mead production. Int J Food Microbiol. 2010 Nov 15; 144(1):193-8*).*

The main objectives of research by *Pereira AP et al. (2009)* were to evaluate the capacity of *Saccharomyces cerevisiae* strains, isolated from honey of the Tras-os-Montes (Northeast Portugal), to produce mead. Five strains from honey, one laboratory strain and one commercial wine strain, were evaluated in terms of their fermentation performance under ethanol, sulphur dioxide and osmotic stress. All the strains showed similar behaviour in these conditions. Two yeasts strains isolated from honey and the commercial wine strain were further tested for mead production, using two different honey (a dark and a light honey), enriched with two supplements (one commercial and one developed by the research team), as fermentation media. The results obtained in this work showed that S. *cerevisiae* strains isolated from honey, are appropriate for mead production. However it is of extreme importance to take into account the characteristics of the honey, and supplements used in the fermentation medium formulation, in order to achieve the best results in mead production (Pereira AP et al. Mead production: selection and characterization assays of Saccharomyces cerevisiae strains. Food Chem Toxicol. 2009 Aug; 47(8): 2057-63).

*Pereira AP et al. (2013)* presented research on optimization of mead fermentation. The results obtained in this study demonstrated that yeast strain and inoculum size can favorably impact mead's flavor and aroma profiles. (Pereira AP et al.High-cell-density fermentation of Saccharomyces cerevisiae for the optimisation of mead production. Food Microbiol. 2013 Feb; 33(1):114-23).

In assessing the quality of dry wine yeast or dry mead yeast, apart from yeast fermentation capacity also their microbiological purity and the number of dead cells are taken into consideration. Premium yeast may be infected with other microorganisms, amongst others: wild yeast, fermentative bacteria, moulds etc. An excessive amount of other microflora may cause a deterioration of organoleptic properties and decrease the shelf life of wine. Whereas with an excessive number of dead yeast cells their fermentation activity may be reduced. Mead yeast may be supplied solely on agar medium or in the form of a liquid starter culture, both forms exclude storage for more than a few weeks subject to being stored in a cold store, and require the starter culture to propagate for a few days. The use of a starter culture in the form of dry yeast guarantees proper fermentation and allows to achieve a product with the required physicochemical and sensoric properties. In the event of using non sterile worts (unboiled, where valuable nutrition components are not destroyed during boiling) dry preparations eliminate the risk of undesired microflora contamination, which is particularly important for large scale production.

Thus the aim of the invention is to develop a method for high sugar honey wort alcohol fermentation with the use of a suitable strain of yeast as well as the delivery of a dry yeast preparation that guarantees appropriate shelf life and retention of process properties, allowing use of the obtained preparations in an efficient fermentation process, with an almost instantaneous metabolism functions activation, both immediately after obtaining the preparation as well as after a few months of storage.

The subject of the invention is a high sugar honey wort alcohol fermentation method such that Scm strain of *Saccharomyces cerevisiae* yeast deposited at the IAFB Collection of Industrial Microorganisms under number KKP 2052 p is introduced into the honey wort mash and the wort fermentation is carried out, preferably for 30 days, until a young mead is obtained.

Preferably, the mash contains more than 20°Balling of real extract.

Preferably, a dried Scm strain of *Saccharomyces cerevisiae* yeast is introduced into the wort mash.

Preferably, dry mass content in the dry *Saccharomyces cerevisiae* Scm strain preparation is 92-94%.

Preferably, the *Saccharomyces cerevisiae* yeast Scm strain dry preparation is obtained by its application to wheat bran and subsequent drying.

Preferably, *Saccharomyces cerevisiae* (Scm) yeast with density of 10⁶ /1 ml in between 40% and 50% per volume is combined with the bran in a weight ratio of 1:10, and the obtained mass is subjected to a drying process during which water evaporates at temperature between 20°C and 40°C until the water content of no less than 10% is obtained.

Another subject of the invention is mead beverage obtained as a result of alcohol fermentation of high sugar honey wort conducted in accordance with the defined method. Another subject of the invention is the use of *Saccharomyces cerevisiae* (Scm) yeast strain deposited at the IAFB Collection of Industrial Microorganisms under number KKP 2052 p for the production of mead.

Preferably, the *Saccharomyces cerevisiae* (Scm) yeast strain is used as a dry preparation.

Another subject of the invention is *Saccharomyces cerevisiae* yeast strain Scm deposited at the IAFB Collection of Industrial Microorganisms under number KKP 2052 p.
Fig. 1. Concentrations of sugars and ethyl alcohol after a) 60 and b) 91 days of fermentation of honey wort with different densities. Mead yeast Scm strain. Determined using HPLC.

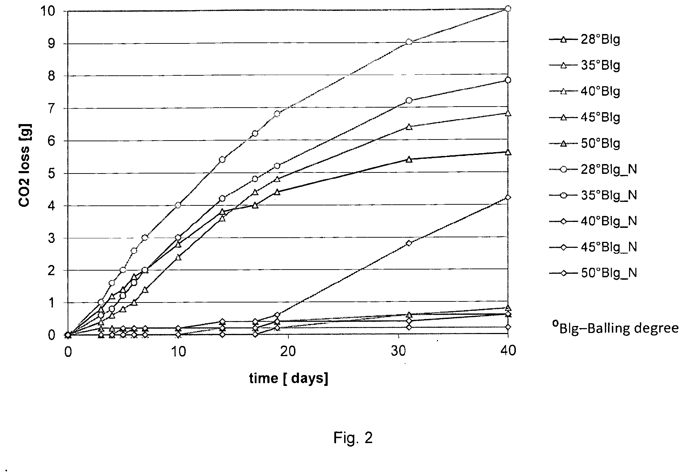

Fig. 2 Loss of CO₂ during honey worts fermentation with different primary sugar concentrations (N - fermentation with addition of nitrogen source).

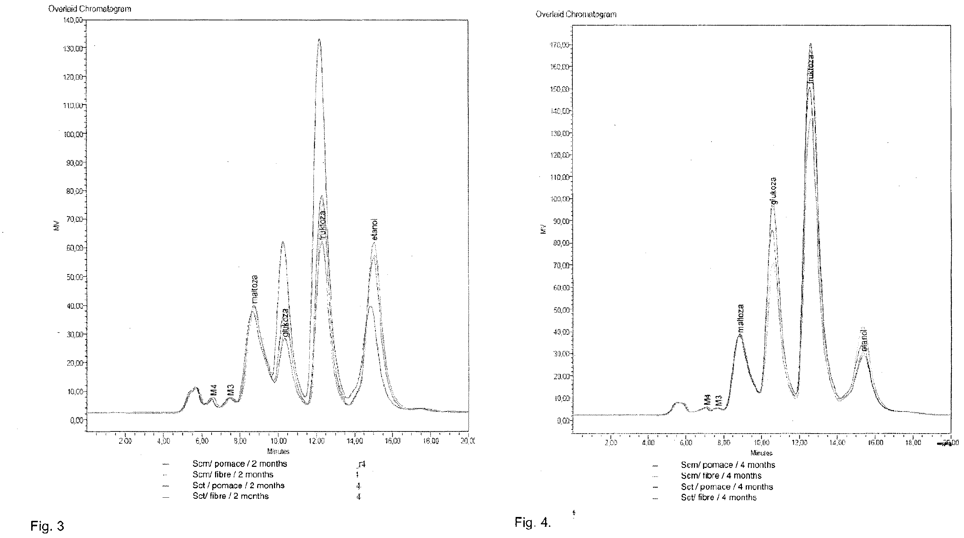

Fig. 3 Chromatograms of mead samples obtained after fermentation with the use of dry yeast stored for 2 months.
Fig. 4 Chromatograms of mead samples obtained after fermentation with the use of dry yeast stored for 4 months.

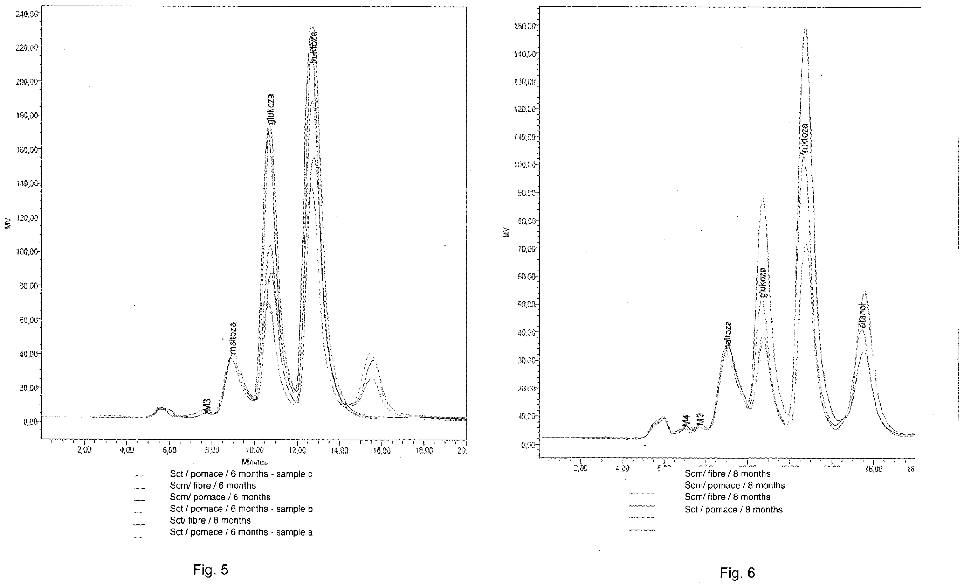

Fig. 5 Chromatograms of mead samples obtained after fermentation with the use of dry yeast stored for 6 months.
Fig. 6 Chromatograms of mead samples obtained after fermentation with the use of dry yeast stored for 8 months.

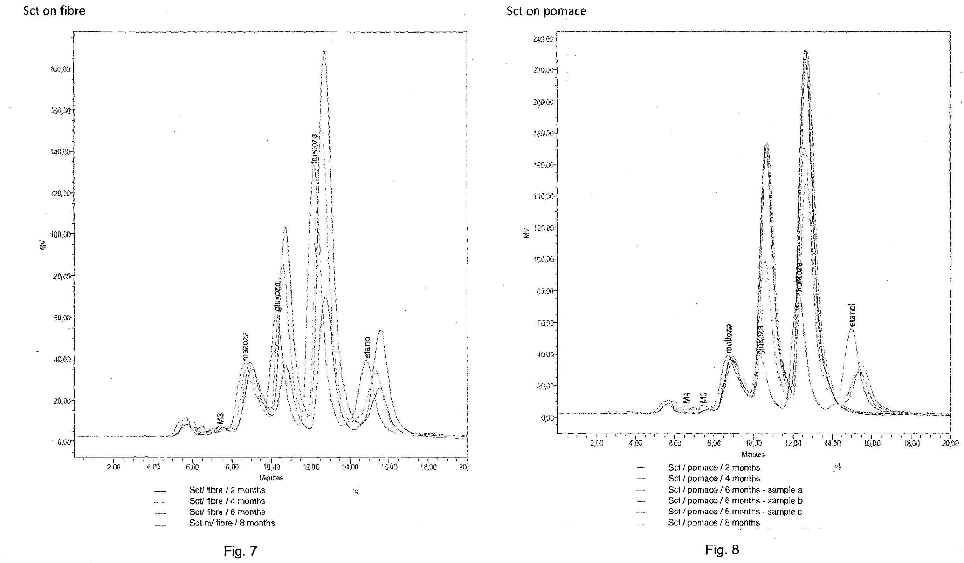

Fig. 7 Chromatograms of mead samples obtained after fermentation with the use of Sct yeast strain dried on bran after various storage times.
Fig. 8 Chromatograms of mead samples obtained after fermentation with the use of Sct yeast strain dried on apple pomace after various storage times.

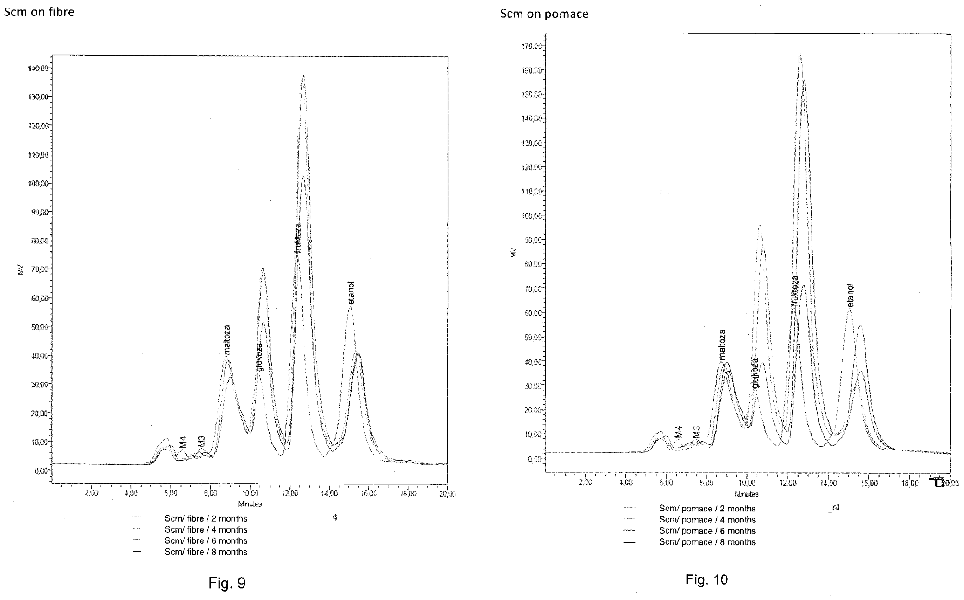

Fig. 9 Chromatograms of mead samples obtained after fermentation with the use Scm yeast strain dried on bran after various storage times.
Fig. 10 Chromatograms of mead samples obtained after fermentation with the use Scm yeast strain dried on apple pomace after various storage periods.
Fig. 11 Glucose and fructose content in meads after 30 day fermentation of honey wort with primary density of 28°Balling. Scm and Sct mead yeast preparations dried on bran or pomace were used as inoculum after different storage periods.
Fig. 12 Ethanol content in meads after 30 day fermentation of honey wort with primary density of 28°Balling. Scm and Sct mead making yeast preparations dried on bran or pomace were used as inoculum after different storage periods. Results were obtained using HPLC method.
Fig. 13 Young mead parameters after 30 day fermentation of honey wort with primary density of 28°Balling. Yeast taken directly from slants were used as inoculum. Results were obtained using HPLC.
Fig. 14 Comparison of young wine and young mead parameters (concentrations of particular sugars and ethyl alcohol) determined with the use of HPLC following 30 day fermentation.
Wine or mead yeast taken directly from slants were used as inoculum.

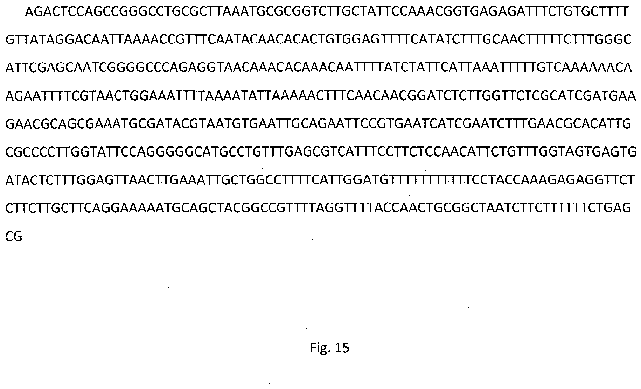

Fig. 15 Nucleotide sequence of ITS1, 5.8S rRNA gene and ITS2 region of *Saccharomyces cerevisiae* strain Scm.

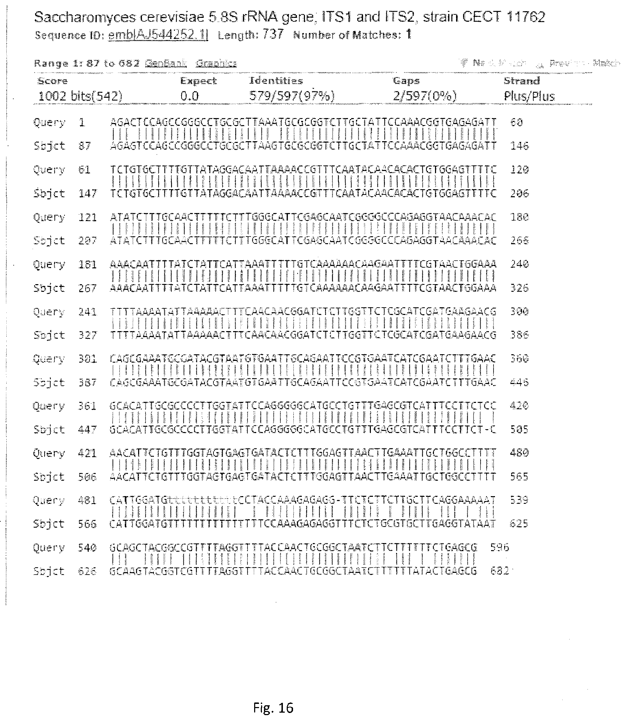

Fig. 16 Comparison of nucleotide sequence of ITS1, 5.8S rRNA gene and ITS2 region of *Saccharomyces cerevisiae* strain Scm with sequences deposited in NCBI.

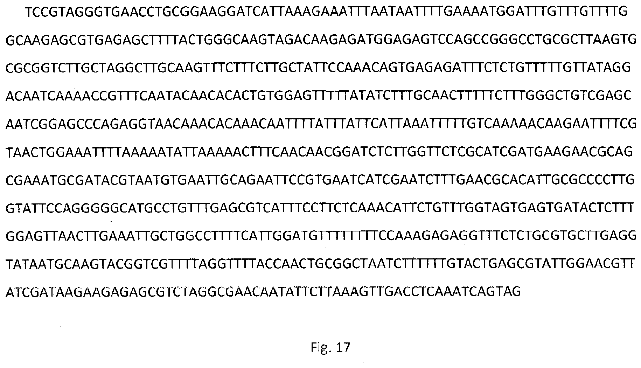

Fig. 17 Nucleotide sequence of partial 18S rRNA gene ITS1, 5.8S rRNA gene and ITS2 and partial 26S rRNA gene of *Saccharomyces cerevisiae* strain Set.

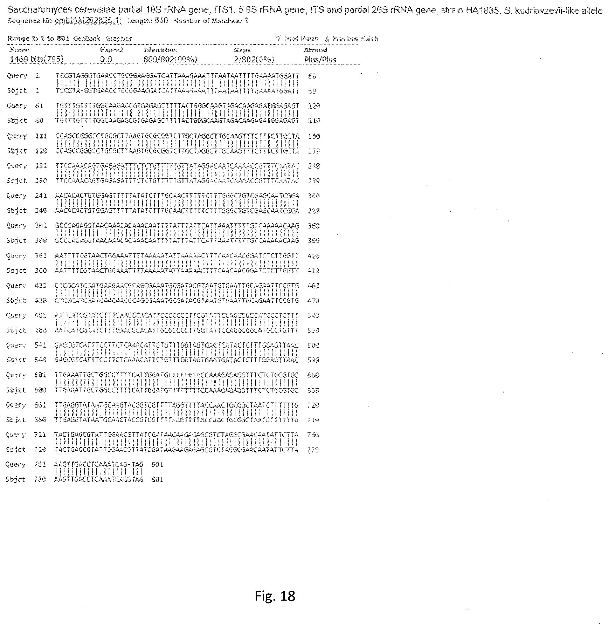

Fig.18 Comparison of nucleotide sequence of partial 18S rRNA gene ITS1, 5.8S rRNA gene and ITS2 and partial 26S rRNA gene of Saccharomyces cerevisiae strain Set with sequences deposited in NCBI.

### Example

### Obtaining yeast preparation

The inoculum was propagated with the use of *Saccharomyces cerevisiae* (Scm). Yeast biomass propagation was carried in stationary conditions in aerated flasks or in fermenter, having capacity up to 5 dm³ with aeration of 1vol./vol. and rotation at 800÷900 rpm. Once cultivation is completed, the yeast was centrifuged, rinsed a number of times with sterile water, mixed with sterile medium of yeast bran or apple pomace and dried. Preparation for drying process is carried out by combining 10⁶ /1 ml density yeast with between 40% and 50% per volume bran in a weight ratio of 1:10. The obtained mass is subjected to a drying process during which water evaporates at temperatures between 20°C and 40°C until water content of no less than 10% is obtained. Dried yeast preparations were stored in a room temperature for periods between a few to a dozen or so months.

### Fermentation tests

Fermentation tests were carried out in 500 ml flat bottom flasks closed off with fermentation tubes containing 250ml apple must or honey wort depending on the type of yeast used.

Dry mead yeast was added to the flasks. Also fermentations were carried out using yeast collected from slant and liquid inoculum, these were treated as control fermentations. Fermentations were carried out at a temperature suitable for the yeast needs. Upon fermentation completion the yeast was separated by centrifuging at 3000 rpm for 15 minutes. Wine yeast culture activity testing entailed determining fermentation energy and young wine characteristics (sugar, alcohol content). Fermentation energy of analysed yeast strains was determined on the basis of CO₂ produced during fermentation.

### Honey mead fermentations at different sugar levels

Honey mead fermentations at different sugar levels were carried out. Mead yeast Scm strain was used as inoculum. Yeast was collected directly from slant, fermentation was carried out at 20°±2°C. Due to different wort densities fermentation was performed for a period of more than 30 days, some of fermentations were stopped after 39 days and other after 60 days. CO₂ reduction during fermentation is shown in table 1. Subsequent tables show "young mead" analyses results obtained using standard methods and chromatography.

**Table 1. Physicochemical properties of "young mead" after 39 and 60 day of fermentation with use of mead yeast, depending on primary wort concentrations.**

| Parameter | Time [days] | Primary wort density [°Balling] | | | | |
|---|---|---|---|---|---|---|
| | | 28 | 35 | 40 | 45 | 50 |
| Alcohol - after 39 days [vol. %] | 39 | 3.0 | 6.6 | 4.2 | 3.0 | 2.0 |
| | 60 | 5.2 | 7.0 | 5.2 | 3.4 | 2.0 |
| Apparent extract [°Balling] | 39 | 22.5 | 27.0 | 35.3 | 43.0 | 48.5 |
| | 60 | 20.0 | 23.0 | 35.0 | 42.0 | 48.0 |
| Real extract [g/100 ml] | 39 | 23.5 | 28.0 | 32.3 | 40.0 | 43.0 |
| | 60 | 22.0 | 25.7 | 36.5 | 43.4 | 49.5 |
| Sugars as sucrose [%] | 60 | 24.45 | 28.21 | 49.76 | 63.61 | 66.69 |
| Acidity [g malic acid/l] | | 2.91 | 3.52 | 4.25 | 3.88 | 3.45 |

The higher the wort concentration the later first CO₂ loss was observed in samples. As wort concentration was increasing, the total CO₂ loses after 39, 60 and 90 days were lower. For 40, 45 and 50 °Balling worts after day 40 mass loss was minimal. In 28 and 35 °Balling worts sample mass changes caused by CO₂ loss were observed even up to day 90. Most of ethyl alcohol after 90 days of fermentation was obtained in worts with primary density of 35°Balling - approximately 7%. In worts with primary density of 28°Balling and 40°Balling after 90 days 5% ethanol was obtained. In 28°Balling wort it would have been possible to attenuate the remaining sugars and obtain higher ethanol concentrations however the fermentation temperature (20°C) was too low.

**Table 2 Young mead parameters after 30 day honey wort fermentation. Saccharomyces cerevisiae (Scm) mead yeast cells were used as inoculum taken directly from slant.**

| Parameter | *Saccharomyces cerevisiae* (Scm) strain |
|---|---|
| Alcohol concentration [vol %] | 12.3 |
| Sugar content expressed as sucrose [%] | 9.96 |
| Overall acidity [g malic acid/l] | 3.62 |
| Total extract [g/l] | 151.06 |

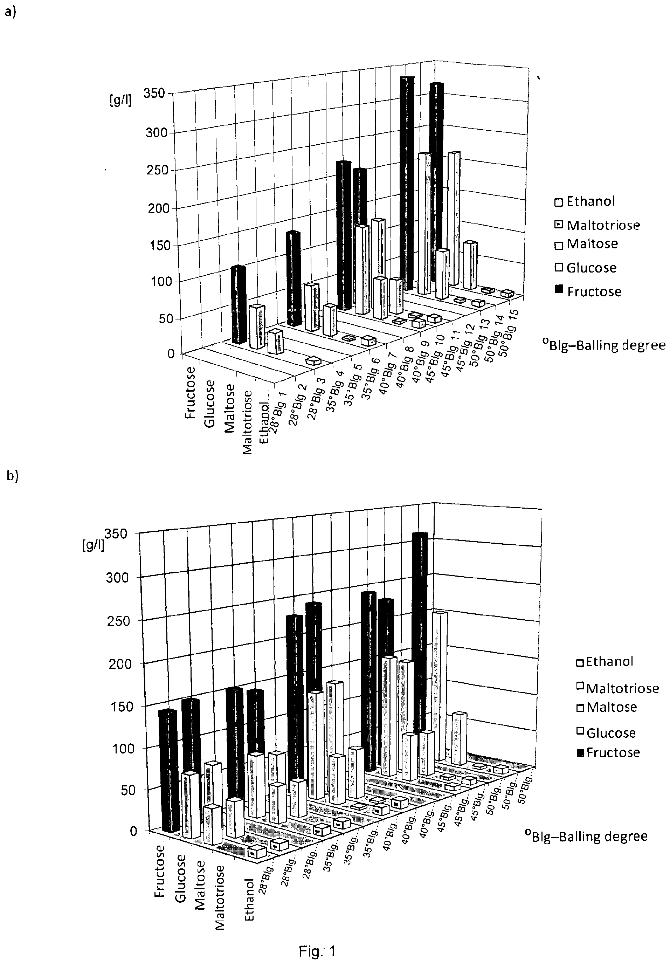

Scm mead yeast was propagated in stationary conditions and 37 g/l was obtained as well as in a fermenter and different worts: honey wort with density 28°Balling and wort based on apple concentrate with density of 21°Balling. Fig. 1 shows HPLC results.

**Table 3. Young mead parameters after 30 day honey wort fermentation. Sct or Scm mead yeast cells were used as inoculum taken directly from slant.**

| Parameter | Strain | |
|---|---|---|
| | Sct | Scm |
| Alcohol concentration [vol %] | 11.3 | 12.3 |
| Sugar content expressed as sucrose [%] | 13.56 | 9.96 |
| Overall acidity [g malic acid/l] | 3.62 | 3.62 |
| Total extract [g/l] | 196.84 | 151.06 |

**Table 4. Young mead parameters after 30 day honey wort fermentation. Sct or Scm dry mead yeast directly after preparing were used as inoculum**

| Parameter | Sct | | Scm | |
|---|---|---|---|---|
| | Pomace | Bran | Pomace | Bran |
| Alcohol concentration [vol %] | 10.3 | 12.2 | 14.7 | 14.3 |
| Sugar content expressed as sucrose [%] | 10.14 | 10.6 | 7.46 | 7.6 |
| Overall acidity [g malic acid/l] | 3.75 | 3.69 | 3.89 | 4.19 |
| Total extract [g/l] | 161.12 | 149.92 | 127.77 | 117.92 |

**Table 5. Young mead parameters after 30 day honey wort fermentation. Sct or Scm mead yeast stored for 2 months at room temperature were used as inoculum**

| Parameter | Sct | | Scm | |
|---|---|---|---|---|
| | Pomace | Bran | Pomace | Bran |
| Alcohol concentration [vol %] | 12.3 | 11.0 | 13.7 | 11.7 |
| Sugar content expressed as sucrose [%] | 9.98 | 13.44 | 10.17 | 10.23 |
| Overall acidity [g malic acid/l] | 3.58 | 3.33 | 3.43 | 3.54 |
| Total extract [g/l] | 141.04 | 170.16 | 131.43 | 142.89 |

**Table 6. Young mead parameters after 30 day honey wort fermentation. Sct or Scm mead yeast stored for 4 months at room temperature were used as inoculum**

| Parameter | Sct | | Scm | |
|---|---|---|---|---|
| | Pomace | Bran | Pomace | Bran |
| Alcohol concentration [vol %] | 6.1 | 7.0 | 6.5 | 9.1 |
| Sugar content expressed as sucrose [%] | 22.6 | 27.0 | 23.1 | 18.8 |
| Overall acidity [g malic acid/l] | 3.45 | 3.15 | 3.25 | 3.43 |
| Total extract [g/l] | - | 242.76 | 248.6 | 206.87 |
| % dry mass | 18.04 | 9.93 | 17.86 | 20.16 |

**Table 7. Young mead parameters after 30 day honey wort fermentation. Sct or Scm mead making yeast stored for 6 months at room temperature were used as inoculum**

| Parameter | Sct | | Scm | |
|---|---|---|---|---|
| | Pomace | Bran | Pomace | Bran |
| Alcohol concentration [vol %] | - | 5.07 | 7.34 | 9.87 |
| Sugar content expressed as sucrose [%] | - | - | - | - |
| Overall acidity [g malic acid/l] | 1.27 | 3.07 | 3.31 | 3.43 |
| Total extract [g/l] | 311.65 | 272.24 | 236.22 | 190.69 |
| % dry mass | 17.44 | 17.80 | 19.04 | 23.33 |

**Table 8. Young mead parameters after 30 day honey wort fermentation. Sct or Scm mead yeast stored for 8 months at room temperature were used as inoculum**

| Parameter | Sct | | Scm | |
|---|---|---|---|---|
| | Pomace | Bran | Pomace | Bran |
| Alcohol concentration [vol %] | 4.4 | 11.9 | 11.8 | 9.5 |
| Sugar content expressed as sucrose [%] | 12.99 | 11.24 | 12.03 | 15.67 |
| Overall acidity [g malic acid/l] | 2.70 | 3.21 | 3.26 | 3.14 |
| Total extract [g/l] | 257.72 | 135.48 | 136.58 | 169.02 |

Once fermentation was stopped, slightly higher concentrations of alcohol and lower extract and sugar concentrations were obtained with the use of Scm strain of yeast, the differences are more pronounced for yeast from slant and preparations used immediately after their preparation as well as preparations stored for 2 months.

### Analysis of differences in ethanol concentrations between given samples and differences in the concentrations of glucose and fructose.

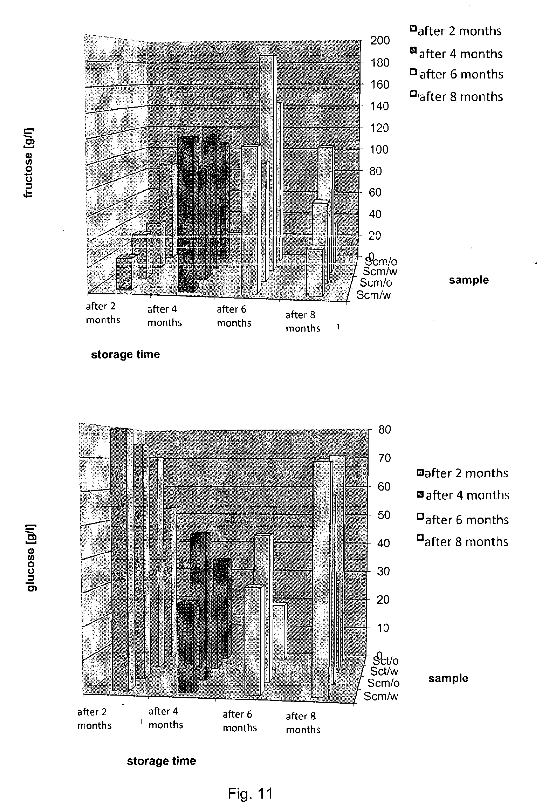

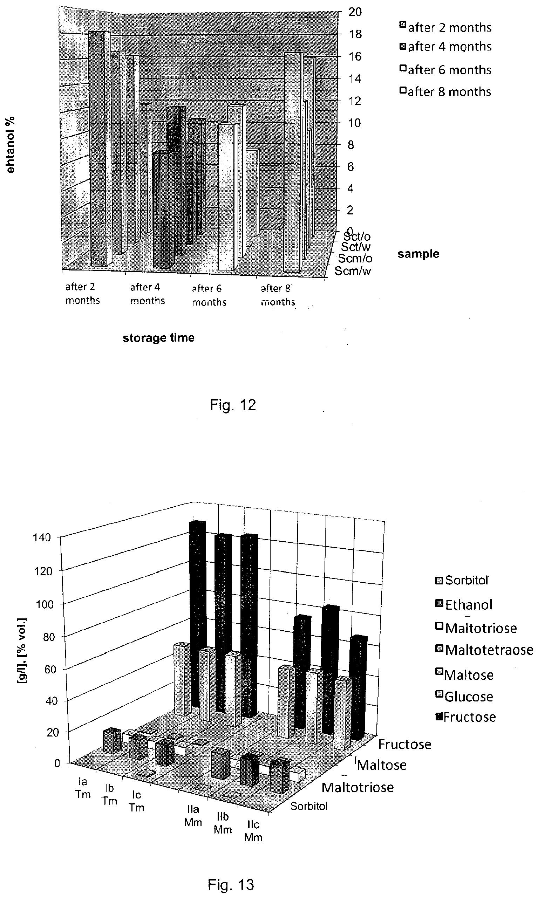

In table 9 and Fig. 11 and 12 glucose, fructose and ethanol concentrations are depicted obtained during HPLC analysis of samples.

**Table 9. Glucose, fructose and ethanol content in meads after 30 day fermentation. Various mead yeasts following different storage periods were used as inoculum. Determined using HPLC.**

| Fructose [g/l] | Sct/b | Sct/p | Scm/b | Scm/p |
|---|---|---|---|---|
| 2 months | 86.186 | 39.177 | 36.617 | 25.260 |
| after 4 months | 105.716 | 123.661 | 92.717 | 120.225 |
| after 6 months | 143.897 | 185.248 | 97.482 | 114.522 |
| after 8 months | 35.909 | 107.588 | 65.895 | 36.476 |

| Glucose [g/l] | Sct/b | Sct/p | Scm/b | Scm/p |
|---|---|---|---|---|
| 2 months | 52.519 | 70.262 | 74.055 | 78.387 |
| after 4 months | 34.419 | 24.345 | 46.079 | 26.562 |
| after 6 months | 19.074 | | 45.889 | 31.763 |
| after 8 months | 70.806 | 29.153 | 58.193 | 68.494 |

| Ethanol [vol %] | Sct/b | Sct/p | Sct/b | Sct/p |
|---|---|---|---|---|
| after 2 months | 11.751 | 16.209 | 16.590 | 18.097 |
| after 4 months | 10.393 | 8.689 | 12.043 | 8.861 |
| after 6 months | 7.709 | 0.000 | 12.216 | 11.088 |
| after 8 months | 15.897 | 9.914 | 12.611 | 16.466 |

During fermentation with the use of yeast stored for 2 months the most ethanol was obtained using fermentation with the use of Scm strain dried on apple pomace. A similar amount of alcohol was obtained during fermentation with the use of Scm strain dried on bran and Sct dried on pomace. The least ethanol was obtained in mead inoculated with Sct yeast on bran. In fermentations with yeast after 4 months of storage the most ethanol appeared in a sample inoculated with Scm yeast on bran, slightly less in a sample inoculated with Sct yeast on bran, and the least (approx. 8%) in samples based on Sct and Scm yeasts on pomace. In samples where less ethanol was obtained glucose and fructose were attenuated to a lesser degree.
In samples with yeast after 6 months of storage most ethanol was obtained in the sample based on bran dried Scm strain (12%), slightly less (11%) was obtained in the pomace dried Scm sample, approx. 7.7% of alcohol was obtained after fermentation with bran dried Sct strain.
After 8 months of storage the most ethyl alcohol was produced by Scm strain yeast on pomace and Sct on bran (∼16%), less ethanol was found in the sample fermented with Scm on bran (∼12.5%) and the least in Sct on pomace sample (∼10%).

During fermentations where bran dried Scm yeast was used, most alcohol was obtained after using a preparation stored for 2 months, similar alcohol amounts, approx. 12 % vol. were obtained in fermentations using preparations stored for 4, 6 and 8 months. Least sugars, glucose and fructose were left in meads in which 2 month preparation was used. Increasingly higher concentrations of sugars were found in meads obtained using preparations stored for 8, 6 and 4 months respectively.

In fermentations where pomace dried Scm yeast was used most ethyl alcohol was obtained using preparations stored for 2 months and then it decreased in wines wherein 8, 6 and 4 (most decreased) month preparations were used. Sugar concentrations after fermentation increased inversely to alcohol concentrations. The least sugars were found after using 2 month preparations and then it increased for 8, 6 and 4 month preparations.

Fig. 13 shows young mead parameters after 30 day fermentation, analysis based on Liquid Chromatography methods. Yeast taken directly from slants were used as inoculum for fermentation. Samples where Scm strain yeast was used as inoculum a slightly better attenuation of sugars was achieved (fructose in the first place followed by glucose) and a slightly higher ethanol concentrations than in samples where Sct yeasts were used.

Concentrations of particular sugars and ethyl alcohol determined using HPLC in young mead after 30 day fermentation. Yeast taken directly from slants were used as inoculum. Scm strain attenuated fructose better and produced more ethyl alcohol than Sct strain. Glucose concentrations after 30 day fermentation is Scm and Sct strains are similar. Fructose and glucose determination results obtained using HPLC are in line with the results obtained by expressing sugars in terms of sucrose.

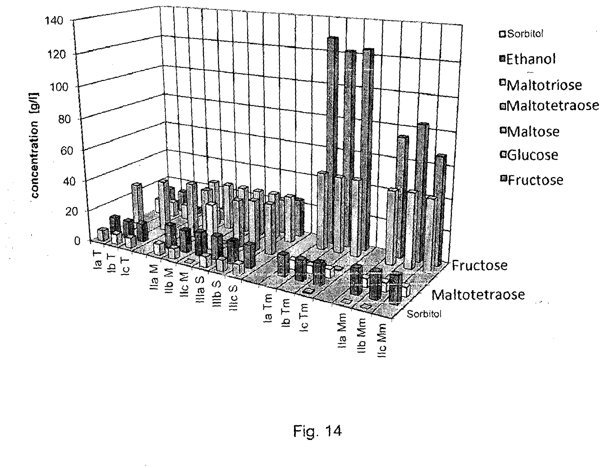

Fig. 14 shows a comparison of young wines and meads.

Ethyl alcohol concentration after 30 day fermentation was similar in young wines and young meads. Sct strains produced slightly less ethanol than Scm strains. In young wine small amounts of sorbitol were found (sorbitol was found in must, very small amounts were produced during fermentation). No sorbitol was found in honey wort or meads after 30 day fermentation. Trisaccharides found in meads were determined to be maltotriose, in wines based on apple must maltotriose was not found after fermentation with the exception of one sample where maltotriose was found but no sorbitol was found. In meads after 30 day fermentation were found many times higher fructose concentrations and glucose concentrations approximately 1.5 time higher that young wines. This primarily stems from the content and density of fermented wort. Scm strains were better than Sct strains at attenuating fructose.

### Fermentations using yeast propagated in a fermenter

Table 10 shows young mead parameters after 30 day honey wort fermentation. Mead yeast propagated in a fermenter were used. Preparations were used directly after being made. Table 10. Young wine parameters after 30 day fermentation. Scm mead making yeast propagated in a fermenter and bran dried were used as inoculum.

| Parameter | |
|---|---|
| Alcohol concentration [vol %] | 10.6 |
| Sugar content expressed as sucrose [%] | 12.2 |
| Overall acidity [g malic acid/l] | 3.99 |
| Total extract [g/100 ml] | 146.57 |

The length of growing yeast in the fermenter did not affect the quality of mead. Ethanol concentrations, general acidity meet the required standards.

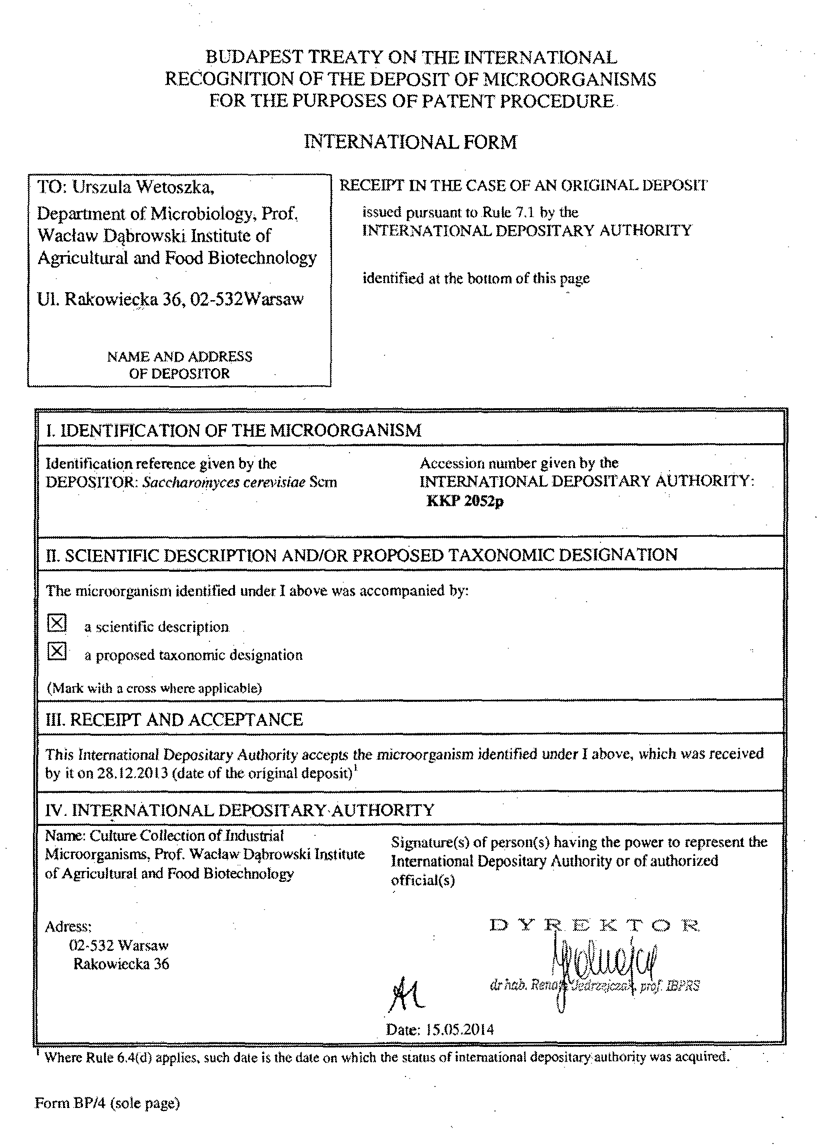

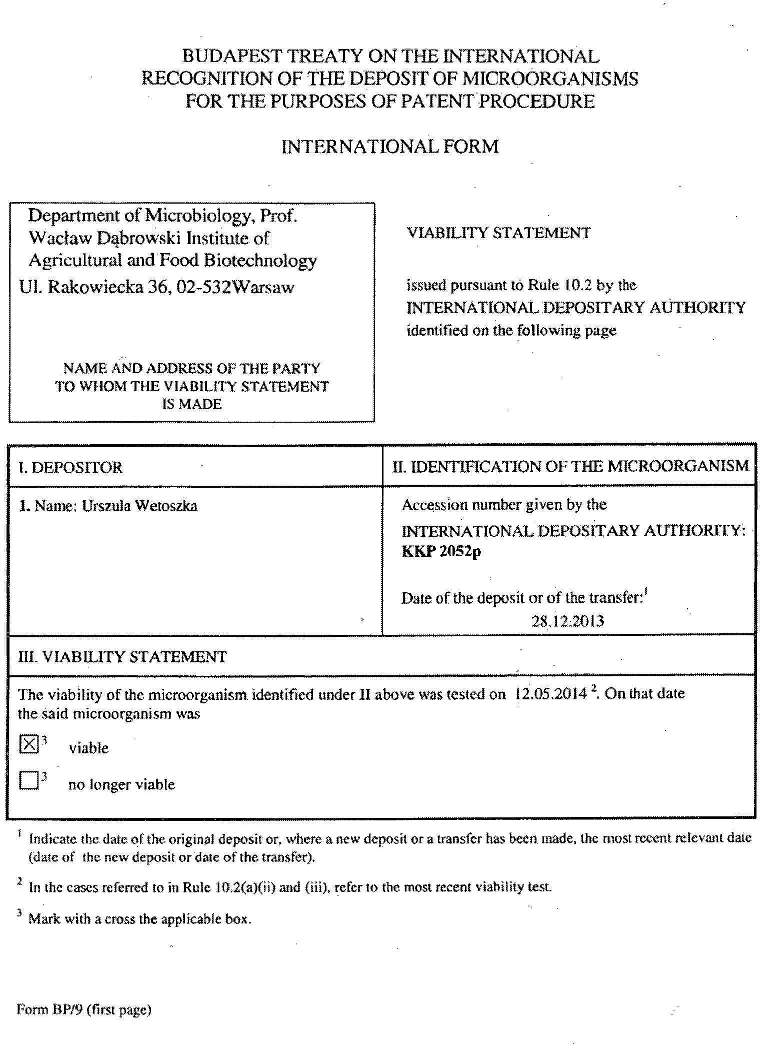

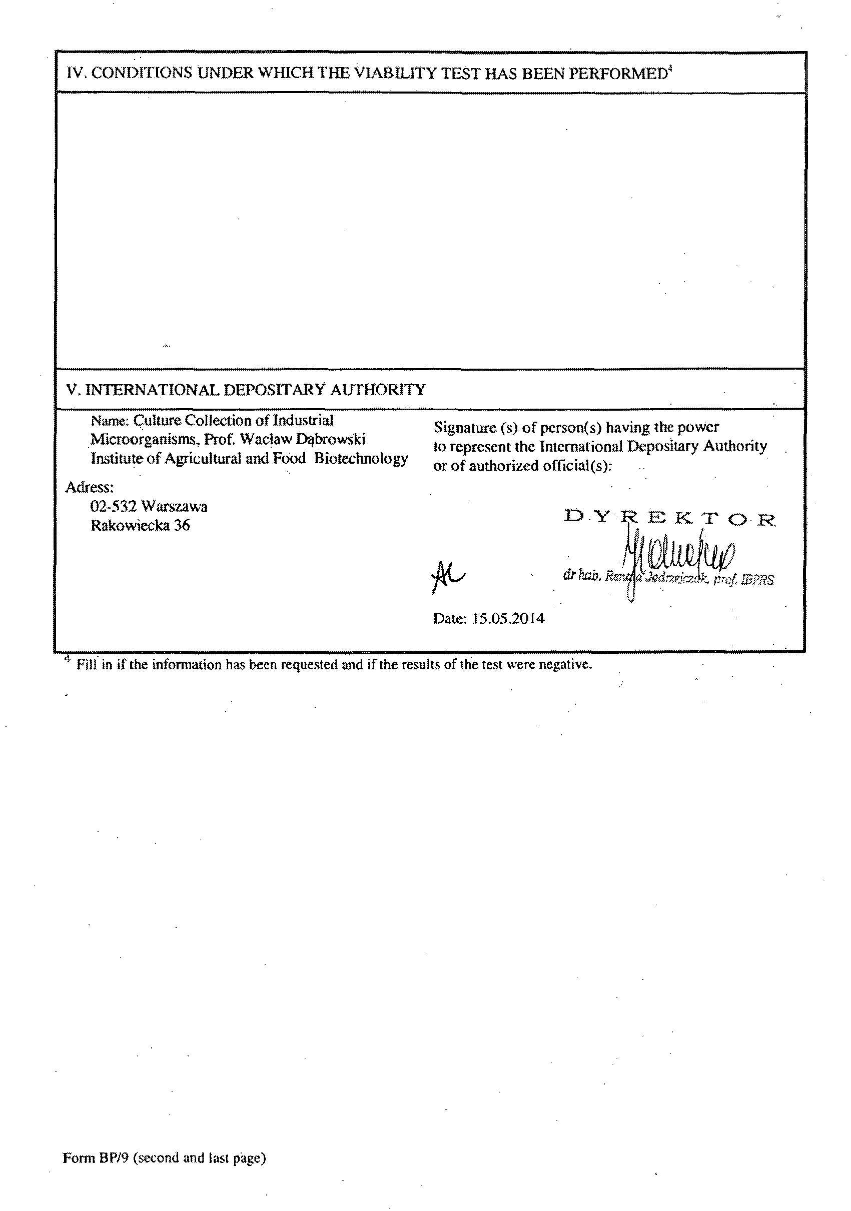

## Claims

1. A method for fermentation of high sugar honey wort wherein Scm strain of *Saccharomyces cerevisiae* yeast deposited at the IAFB Collection of Industrial Microorganisms under number KKP 2052 p is introduced into the honey wort mash and the wort fermentation is carried out, preferably for 30 days, until a young mead is obtained.

2. The method according to claim 1, **characterised in that** the mash contains more than 20°Balling of real extract.

3. The method according to claim 1, **characterised in that** a dried Scm strain of *Saccharomyces cerevisiae* yeast is introduced into the wort mash.

4. The method according to claim 3, **characterised in that** a dried mass content in dry *Saccharomyces cerevisiae* Scm strain preparation is 92-94%.

5. The method according to claim 3, **characterised in that** the *Saccharomyces cerevisiae* yeast Scm strain dry preparation is obtained by its application to wheat bran and subsequent drying.

6. The method according to claim 5, **characterised in that** a *Saccharomyces cerevisiae* (Scm) yeast with density of 10⁶ /1 ml in between 40% and 50% per volume is combined with the bran in a weight ration of 1:10, and the obtained substance is subjected to a drying process during which water evaporates at temperatures between 20°C and 40°C until water content of no more than 10% is obtained.

7. *Saccharomyces cerevisiae* yeast strain Scm deposited at the IAFB Collection of Industrial Microorganisms under number KKP 2052 p.

8. The use of *Saccharomyces cerevisiae* (Scm) yeast strain deposited at the IAFB Collection of Industrial Microorganisms under number KKP 2052 p for the production of mead.

9. The use according to claim 8 **characterised in that** the *Saccharomyces cerevisiae* (Scm) yeast strain is used as a dry preparation.

## Patentansprüche

1. Ein Verfahren zur Fermentation von Hochzucker-Honigwürze, wobei der bei der IAFB-Kollektion industrieller Mikroorganismen unter Nummer KKP 2052 p hinterlegte Sem-Stamm von *Saccharomyces cerevisiae* in die Honigwürzemaische eingebracht wird und die Honigwürzefermentation, vorzugsweise über 30 Tage, ausgeführt wird, bis eine junger Honigwein erhalten wird.

2. Das Verfahren nach Anspruch 1, dadurch charakterisiert, dass die Maische mehr als 20°Balling echten Extrakts enthält.

3. Das Verfahren nach Anspruch 1, dadurch charakterisiert, dass ein getrockneter Sem-Stamm von *Saccharomyces cerevisiae* in die Würzmaische eingebracht wird,

4. Das Verfahren nach Anspruch 3, dadurch charakterisiert, dass der Trockenmassegehalt in der trockenen *Saccharomyces* cerevisiae-Stammpräparation 92-94% ist.

5. Das Verfahren nach Anspruch 3, dadurch charakterisiert, dass die *Saccharomyces cerevisiae-*Hefestammpräparation durch ihre Applikation an Weizenkleie und anschließende Trocknung erhalten wird.

6. Das Verfahren nach Anspruch 5, dadurch charakterisiert, dass eine *Saccharomyces cerevisiae* (Scm)-Hefe mit einer Dichte von 10⁶ / 1 ml in zwischen 40% und 50% pro Volumen mit der Kleie in einem Gewichtsverhältnis von 1:10 kombiniert wird, und die erhaltene Substanz einem Trocknungsprozess unterworfen wird, während dessen Wasser bei Temperaturen zwischen 20 °C und 40 °C verdampft, bis ein Wassergehalt von nicht mehr als 10% erhalten wird.

7. Der bei der IAFB-Kollektion industrieller Mikroorganismen unter Nummer KKP 2052 p hinterlegte *Saccharomyces* cerevisiae-Hefestamm Scm.

8. Die Verwendung des bei der IAFB-Kollektion industrieller Mikroorganismen unter Nummer KKP 2052 p hinterlegten Hefestamms von *Saccharomyces cerevisiae* (Scm) zur Herstellung von Honigwein.

9. Die Verwendung nach Anspruch 8, dadurch charakterisiert, dass der Hefestamm von *Saccharomyces cerevisiae* (Scm) als Trockenpräparat verwendet wird.

## Revendications

1. Procédé de fermentation d'un moût de miel à haute teneur en sucre dans lequel une souche Scm de levure *Saccharomyces cerevisiae* déposée auprès de la Collection des micro-organismes industriels IAFB sous le numéro KKP 2052 p est introduite dans la maische de moût de miel et la fermentation de moût est réalisée, de préférence pendant 30 jours, jusqu'à ce qu'un hydromel jeune soit obtenu.

2. Procédé selon la revendication 1, **caractérisé en ce que** la maische contient plus de 20 °Balling d'extrait réel.

3. Procédé selon la revendication 1, **caractérisé en ce qu'**une souche Scm sèche de levure *Saccharomyces cerevisiae* est introduite dans la maische de moût.

4. Procédé selon la revendication 3, **caractérisé en ce que** la teneur en masse sèche dans une préparation à base de souche Scm *Saccharomyces cerevisiae* est de 92 à 94 %.

5. Procédé selon la revendication 3, **caractérisé en ce que** la préparation sèche de souche Scm de levure *Saccharomyces cerevisiae* est obtenue par son application sur du son de blé puis par séchage.

6. Procédé selon la revendication 5, **caractérisé en ce qu'**une levure *Saccharomyces cerevisiae* (Scm) d'une densité de 10⁶/1 ml dans entre 40 % et 50 % par volume est combinée avec le son selon un rapport pondéral de 1:10, et la substance obtenue est soumise. à un processus de séchage pendant lequel de l'eau s'évapore à des températures comprises entre 20 °C et 40 °C jusqu'à ce qu'une teneur en eau inférieure ou égale à 10 % soit obtenue.

7. Souche Scm de levure *Saccharomyces cerevisiae* déposée auprès de la Collection des micro-organismes industriels IAFB sous le numéro KKP 2052 p.

8. Utilisation d'une souche de levure (Scm) *Saccharomyces cerevisiae* déposée auprès de la Collection des micro-organismes industriels IAFB sous le numéro KKP 2052 p pour la production d'hydromel.

9. Utilisation selon la revendication 8, **caractérisée en ce que** la souche de levure (Scm) *Saccharomyces cerevisiae* est utilisée sous forme d'une préparation sèche.
